# FASCICULE DE BREVET EUROPEEN

(11) **EP 0 622 055 B1**
(45) Date de publication et mention de la délivrance du brevet: **23.06.1999**
(21) Numéro de dépôt: 94400842.4
(22) Date de dépôt: 18.04.1994
(51) Int. Cl.: A61D 19/00, A61M 1/00

(54) **Dispositif collecteur filtrant d'un liquide visqueux**
Sammel- und Filtervorrichtung für eine viskose Flüssigkeit
Collection and filter device for a viscous liquid

(30) Priorité: 26.04.1993 FR 9304904
(43) Date de publication de la demande: 02.11.1994
(73) Titulaire: INSTRUMENTS DE MEDECINE VETERINAIRE, F-61300 L'Aigle (FR)
(72) Inventeur: Cassou, Robert, F-18700 Aubigny Sur Nère (FR); Cassou, Maurice, F-61400 Mortagne au Perche (FR); Cassou, Bertrand, F-61300 L'Aigle (FR); Brillard Jean-Pierre, F-37300 Joué lès Tours (FR); Raulic Laurent, F-22460 Saint-Hervé (FR)
(74) Mandataire: Obolensky, Michel

(56) Documents cités:
- EP-A- 0 358 302
- FR-A- 2 520 193
- FR-A- 2 578 414
- US-A- 3 872 869
- US-A- 4 083 706
- US-A- 4 402 687

## Description

La présente invention est relative aux dispositifs collecteurs de liquide séminal chez les animaux et se rapporte plus particulièrement à un dispositif collecteur du type à aspiration par dépression.

La collecte du liquide séminal par exemple aviaire se fait jusqu'à présent à l'aide d'un récipient collecteur muni d'un entonnoir au-dessus duquel un opérateur procède à la manipulation de l'animal.

On connaît également d'après le brevet français FR-A-2 520 193, déposé le 26 janvier 1982, déposé aux noms de Messieurs Robert, Maurice et Bertrand CASSOU, un dispositif collecteur à dépression constitué d'un capuchon souple coiffant un récipient collecteur, le capuchon souple étant pourvu d'un orifice central dans lequel est montée une canule d'aspiration du liquide séminal et d'un orifice latéral dans lequel est engagé un tube de création d'une dépression dans le récipient, ce tube étant relié à une pompe ou à un embout d'aspiration buccal.

Ces dispositifs connus présentent l'inconvénient essentiel de recueillir dans le récipient collecteur un produit contenant des corps étrangers tels que poussières, débris de plumes, particules d'aliments, grains de sable, fientes, plus ou moins contaminants, notamment lorsque l'utilisateur exerce des massages trop rapides voire même violents.

Par ailleurs, en raison des impuretés contenues notamment dans le liquide séminal, ces dispositifs connus ne permettent pas l'adjonction dans le récipient collecteur de produits stériles de conservation de la semence.

L'invention vise à remédier aux inconvénients des dispositifs connus en créant un dispositif collecteur de liquide séminal qui permette d'obtenir dans le récipient collecteur un liquide séminal dépourvu de toute impureté et prêt par conséquent à être mélangé à un produit ou dilueur conservateur.

Elle a donc pour objet un dispositif collecteur d'un liquide visqueux tel qu'un liquide séminal comprenant un récipient collecteur sur lequel est monté un embout pourvu d'une canule d'aspiration du liquide sous l'effet d'une dépression, caractérisé en ce qu'il comporte en outre un manchon adaptateur interposé entre le récipient et l'embout, ledit manchon comprenant un filtre en communication avec l'embout d'une part et avec le récipient collecteur d'autre part et un canal d'aspiration destiné à être relié à une source de dépression pour engendrer dans le récipient une dépression destinée à provoquer l'aspiration du liquide à travers ledit filtre.

L'invention a également pour objet un appareil collecteur d'un liquide visqueux tel qu'un liquide séminal, caractérisé en ce qu'il comporte un dispositif collecteur de liquide suivant l'objet mentionné ci-dessus pourvu d'un canal d'aspiration destiné à être relié à une source de dépression, le dispositif collecteur étant engagé de façon amovible dans un boîtier au moyen d'une bague d'adaptation fixée dans une paroi du boîtier et comportant des moyens de liaison étanche du canal d'aspiration du dispositif collecteur avec une source de dépression, comprenant une pompe et un moteur électrique d'actionnement de celle-ci également contenus dans le boîtier, le moteur électrique étant relié à une batterie d'alimentation tandis que la pompe est reliée par un conduit à la bague d'adaptation.

L'invention sera mieux comprise à l'aide de la description qui va suivre, donnée uniquement à titre d'exemple et faite en se référant aux dessins annexés, sur lesquels :
- la Fig.1 est une vue en élévation et en coupe d'un premier mode de réalisation du dispositif collecteur de liquide séminal selon l'invention;
- la Fig.2 est une vue en élévation et en coupe d'un second mode de réalisation du dispositif collecteur filtrant pour un liquide séminal selon l'invention;
- la Fig.3 est une vue en élévation et en coupe d'un troisième mode de réalisation du dispositif collecteur de liquide séminal selon l'invention;
- la Fig.4 est une vue schématique en perspective d'un dispositif collecteur de liquide séminal du type électrique et portable; et
- la Fig.5 est une vue partielle en coupe de la bague d'adaptation du collecteur de la Fig.4.

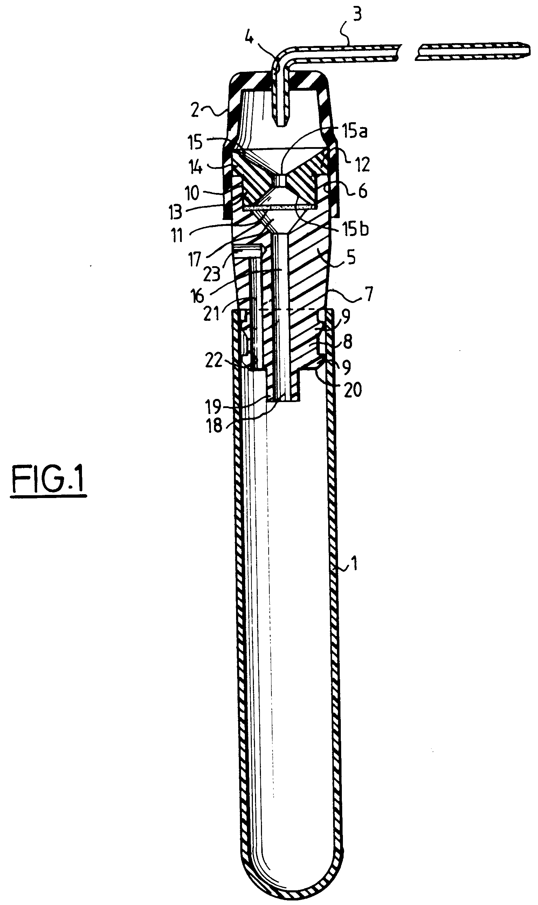

Le dispositif collecteur filtrant pour un liquide séminal représenté à la Fig.1 comporte principalement un récipient collecteur 1 constitué par un tube à essai sur lequel est monté un embout 2 constitué par un capuchon souple en matière plastique pourvu d'une canule d'aspiration ou tube de récolte 3, engagée dans un trou axial 4 du capuchon souple.

Entre le capuchon 2 et le tube 1 est interposé un manchon adaptateur 5 qui comporte une surface latérale extérieure cylindrique 6 de réception de l'extrémité ouverte du capuchon souple 2, prolongée par une surface latérale extérieure 7 légèrement tronconique dont la fonction sera expliquée par la suite.

A son extrémité opposée au capuchon souple 2, destinée à être engagée dans le tube à essai 1, le manchon adaptateur 5 comporte une portion 8 pourvue de nervures périphériques 9 de sertissage dans le tube à essai, inclinées dans la direction de l'introduction du manchon dans le tube et destinées de ce fait à empêcher le retrait du manchon après son engagement.

A son extrémité voisine du capuchon souple 2, le manchon d'adaptation comporte une chambre 10 ouverte vers le capuchon souple 2 et dans laquelle est monté un filtre 11 pour le liquide séminal.

Le filtre 11 est du type à maille très fine de dimension inférieure à 100µm pour filtrer des substances ou liquides moins fluides que l'eau. La dimension des mailles du filtre est adaptée au produit à filtrer.

Le filtre est avantageusement un filtre en polyester ou en polyamide monofil donc sans peluches ou fibres qui risqueraient de se mélanger au liquide séminal.

La matière du filtre est non mouillable et non toxique.

Dans l'exemple représenté à la Fig.1, le filtre 11 est maintenu dans la chambre 10 par une douille de fixation 12 qui comporte une jupe 13 en contact avec la surface latérale de la chambre 10 et appliquant le bord du filtre contre le fond de ladite chambre et un rebord 14 de diamètre égal au diamètre extérieur de la surface latérale 6 du manchon 5. Le rebord 14 définit intérieurement un réceptacle évasé 15 pour la semence, s'étendant immédiatement au-dessous de l'extrémité de la canule d'aspiration 3 engagée dans le capuchon souple 2 et aboutissant à un étranglement cylindrique 15a qui communique à son tour avec une chambre tronconique 15b assurant un élargissement de l'étranglement 15a en vue de répartir le liquide sur la totalité de la surface utile du filtre 11. L'étranglement 15a empêche l'air de passer en même temps que le liquide en dépit des modifications d'inclinaison du dispositif collecteur pendant son utilisation.

Dans le manchon adaptateur 5 est ménagé un conduit axial 16 d'aspiration du liquide séminal qui comporte une portion d'extrémité évasée 17 communiquant avec la chambre 15b à travers le filtre 11 et une portion d'extrémité 18 débouchant dans le tube à essai 1 par une saillie ou bec 19 s'étendant au-delà de l'extrémité 20 du manchon 5 intérieure au tube à essai.

Dans le manchon 5 est en outre ménagé un canal coudé 21 de mise en communication de l'intérieur du tube à essai avec une source de dépression non représentée. Le canal 21 comporte une portion axiale 22 parallèle au conduit d'aspiration 16 qui débouche dans l'extrémité 20 du manchon adaptateur 5 intérieure au tube à essai, c'est à dire en retrait de la portion d'extrémité 18 du conduit 16 d'aspiration du liquide séminal et une portion radiale 23 qui débouche dans la surface latérale du manchon adaptateur 5, de manière à être mise en communication avec la source de dépression non représentée.

Grâce à cet agencement, lorsqu'une dépression est créée à l'intérieur du tube à essai 1 par aspiration à travers le canal 21, cette dépression se traduit par une aspiration à partir du bas du manchon d'adaptation 5 à travers le filtre 11, l'intérieur du capuchon souple 2 et la canule d'aspiration 3.

Le fait que l'extrémité 18 du conduit d'aspiration 16 du liquide séminal fasse saillie par rapport à l'extrémité du canal d'aspiration 21 relié à la source de dépression, empêche qu'une partie du liquide aspiré par le conduit 16 ne pénètre dans le canal d'aspiration 21 relié à la source de dépression.

Le capuchon souple 2 qui est réalisé en une matière transparente, permet un contrôle visuel de la récolte du liquide séminal.

Grâce à la présence du filtre 11, le liquide recueilli est débarrassé de tous corps étrangers tels que poussières, débris de plumes, particules d'aliments, grains de sable, plus ou moins contaminants et surtout fientes dans le cas de la récolte de semence aviaire.

La douille 12 de fixation du filtre 11 oriente la semence sur le filtre et la portion d'extrémité élargie 17 du conduit d'aspiration 16 dont l'extrémité en contact avec le filtre 11 présente une section pratiquement égale à la surface utile de celui-ci, permet de filtrer la semence sur une plus large surface de filtre, qui de ce fait augmente le nombre de doses filtrées.

Le filtre 11 peut être changé rapidement entre deux récoltes sans lourde manipulation. Selon une variante, il peut aussi être rendu solidaire de la chambre de filtration 10 par exemple par collage ou soudage afin d'éviter de le souiller avec les doigts dans le cas d'utilisation d'un manchon d'adaptation ou de filtration entièrement jetable.

Comme indiqué précédemment, le dispositif collecteur de liquide séminal décrit en référence à la Fig.1 équipé de son manchon d'adaptation et de filtration peut être connecté à un système d'aspiration buccal ou électrique.

La chambre de filtration 15b délimitée par la jupe 13 de la bague 12 présente un volume relativement faible permettant ainsi à la semence animale de couvrir plus rapidement la zone de filtration du filtre 11. L'air ne pouvant plus passer à travers le filtre, en raison de la présence de l'étranglement 15a, commence alors la filtration de la semence qui sera orientée vers le tube à essai 1 dans lequel le vide a été créé par aspiration de la manière décrite précédemment.

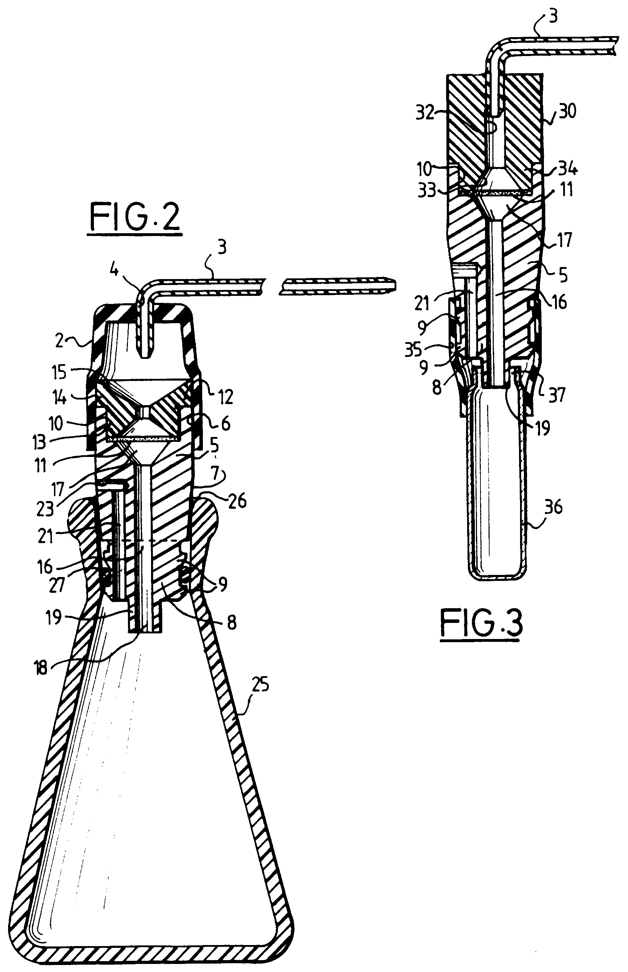

Le dispositif collecteur de liquide séminal représenté à la Fig.2, est en tout point semblable à celui décrit en référence à la Fig.1 à l'exception du fait que le récipient collecteur de ce dernier est constitué par un Erlenmeyer 25.

Afin de permettre le montage étanche du manchon adaptateur 5 dans le col 26 du récipient 25, le manchon est engagé dans le col 26 de façon que sa surface latérale tronconique 7 soit en contact avec la surface tronconique complémentaire du col 26. Un joint torique 27 est fixé sur l'extrémité du manchon 8 comportant les nervures 9 et engagé dans une gorge ménagée entre deux de ces nervures.

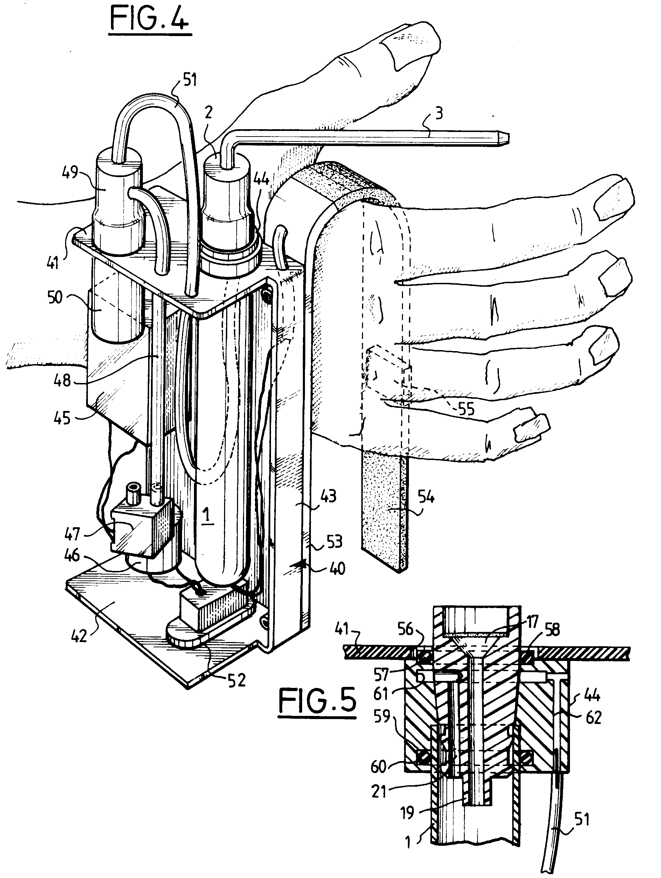

Le dispositif collecteur de liquide séminal représenté à la Fig.3 diffère de ceux décrits en référence aux Fig.1 et 2 en ce qu'à la place du capuchon souple 2 portant la canule d'aspiration 3 des modes de réalisation précédents, il comporte un capuchon rigide 30 réalisant à lui seul les fonctions du capuchon souple 2 et de la douille 12 de fixation du filtre du mode de réalisation de la Fig.1.

Il est constitué par une pièce massive au centre de laquelle débouche un canal 32 dans lequel est engagée la canule d'aspiration 3.

A son extrémité opposée, le canal axial 32 débouche dans une chambre tronconique 33 située juste au-dessus du filtre 11 placé dans la chambre axiale 10 du manchon adaptateur 5. La chambre 33 assure un élargissement du canal 32 en vue de répartir le liquide sur la totalité de la surface utile du filtre. Le canal 32 constitue un étranglement empêchant l'air de passer en même temps que la semence en dépit des modifications d'inclinaison du dispositif collecteur pendant son utilisation.

Le capuchon rigide 30 comporte autour de la chambre tronconique 33 une jupe 34 qui sert à l'immobilisation du filtre 11.

Le manchon d'adaptation 5 est de construction identique à celle des manchons des modes de réalisation des Fig.1 et 2.

Son extrémité 8 pourvue des nervures annulaires 9 reçoit un manchon souple 35 de liaison avec un récipient tubulaire de faible section tel qu'une ampoule précassée 36 qui assure dans ce cas la fonction de récipient collecteur. La saillie 19 du manchon d'adaptation 5 est engagée dans le col de l'ampoule précassée 36 avec un jeu 37 qui permet de créer dans le récipient la dépression nécessaire à l'aspiration au moyen du canal d'aspiration axial 21 relié à une source de dépression non représentée analogue à celle évoquée dans le cadre de la description des modes de réalisation précédents.

On voit que dans le mode de réalisation de la Fig.3, la saillie ou bec 19 en coopération avec le col relativement étroit de l'ampoule précassée 36 empêche la remontée de la semence par le canal d'aspiration 21.

Le manchon souple 35 s'adapte de façon étanche d'une part sur l'extrémité 8 du manchon d'adaptation 5 et d'autre part, sur le pourtour de l'ampoule précassée 36 légèrement au-dessous de son col.

Dans le présent mode de réalisation, la semence est filtrée plus rapidement en raison de la présence du canal étroit 32 dans le bouchon rigide 30 qui supprime le passage de l'air entre la semence et les bords de ce conduit, même lorsqu'il faut aspirer et filtrer une substance épaisse et visqueuse qui adhère aux parois.

Le remplissage de la chambre de filtration se fait également plus rapidement, de sorte que le débit du système de filtration est accru.

Par ailleurs, le capuchon rigide 30 dont le canal axial 32 est connecté directement au conduit d'aspiration 16 du manchon d'adaptation 5, ne présente aucun désamorçage de l'arrivée de la semence dans la chambre de filtration quel que soit l'angle de la canule d'aspiration de semence 3 du dispositif collecteur.

On voit donc que grâce à l'agencement qui vient d'être décrit, le manchon d'adaptation ou de filtration 5 est conçu pour s'adapter sur plusieurs types de récipients collecteurs de semence.

Ainsi, ce manchon 5 constitue une pièce polyvalente du dispositif suivant l'invention.

On va maintenant se référer à la Fig.4 sur laquelle on a représenté en perspective un appareil électrique portable et autonome collecteur de liquide séminal suivant l'invention.

Cet appareil dont le couvercle de protection a été retiré pour plus de clarté, comporte un corps 40 en matériau isolant qui comporte deux parois d'extrémité 41,42 s'étendant perpendiculairement à une embase 43. Dans l'une des parois d'extrémité 41 du corps 40, est montée une bague 44 d'adaptation amovible d'un dispositif collecteur à filtration pour liquide tel que celui décrit en référence à la Fig.1 ou à la Fig.3.

Dans le corps 40 sont en outre montées une batterie rechargeable 45 par exemple de 6 à 9 Volts, connectée à un moteur électrique 46 d'actionnement d'une pompe à dépression 47 reliée par un conduit 48 à un capuchon souple 49 qui coiffe un récipient décanteur 50 et qui est à son tour relié par un conduit souple 51 à la bague d'adaptation 44 d'une manière qui sera décrite en référence à la Fig.5.

Sur la paroi d'extrémité 42 opposée à celle portant la bague d'adaptation 44, le boîtier 40 porte une prise électrique 52 de connexion à un chargeur de batterie non représenté.

Sur la face de l'embase 43 du corps 40 opposé aux côtés d'extrémité 41,42, est montée une poignée 53 en forme de U ou crosse qui porte sur sa branche libre 54 un interrupteur 55 de commande du moteur 46 d'entraînement de la pompe à dépression 47, ledit interrupteur pouvant être actionné par les doigts de la main de l'utilisateur alors que le dos de cette main est en contact avec le fond 43 du boîtier 40 de la manière représentée à la Fig.5.

La présence de la poignée 53 en forme de crosse permet de libérer les cinq doigts de la main afin de procéder au massage des animaux tout en disposant d'un système d'aspiration pour récolter le liquide séminal aviaire.

Un tel dispositif est particulièrement léger et autonome et ne présente ni fil électrique, ni tuyauterie encombrante.

Le vase de décantation 50 qui jouxte le tube collecteur 1 évite que toute impureté ou liquide étranger puisse nuire au bon fonctionnement de la pompe 47.

La bague d'adaptation 44 va maintenant être décrite en référence à la Fig.5.

Elle est montée dans la paroi d'extrémité 41 du boîtier 40 par un rebord axial 56 dans lequel est ménagée une première gorge annulaire 57 recevant un premier joint torique 58. A son extrémité opposée au joint torique 58, la bague 44 comporte une deuxième gorge annulaire 59 dans laquelle est monté un deuxième joint torique d'étanchéité 60.

Entre les deux joints toriques 58 et 60, est ménagée une chambre annulaire 61 dans laquelle débouche un conduit d'aspiration 62 relié au conduit souple 51 de liaison avec la pompe d'aspiration par l'intermédiaire du décanteur 50.

Lorsque le dispositif collecteur représenté à la Fig.1 est monté de façon amovible dans la bague d'adaptation 44 de l'appareil de la Fig.4, la paroi latérale extérieure du manchon d'adaptation 5 est disposée dans la bague 44 de manière que les joints toriques 58 et 60 de celle-ci constituent avec cette paroi latérale un espace étanche reliant l'orifice 23 du canal d'aspiration du manchon d'adaptation 5 avec la chambre annulaire 61 et le canal d'aspiration 62 de la bague 44, ce qui permet lors de la mise en route de la pompe de créer dans le récipient collecteur 1, la dépression nécessaire à l'aspiration du liquide séminal par l'intermédiaire de la canule d'aspiration 3, du filtre 11, et du conduit d'aspiration 16 ménagé dans le manchon d'adaptation 5.

Lorsqu'une opération de collecte dans un dispositif collecteur est terminée, celui-ci est retiré de l'appareil et remplacé par un autre sans nécessiter des débranchements à la main qui risqueraient d'occasionner des souillures source de contamination.

Bien entendu, le tube à essai peut contenir un produit de dilution ou de conservation du liquide séminal permettant une auto-dilution.

Les pièces du dispositif collecteur suivant l'invention ont été conçues de façon à être entièrement moulables et peuvent par conséquent être considérées comme des produits consommables évitant des nettoyages et des stérilisations quotidiens.

On voit que grâce à l'agencement décrit en référence aux Fig.4 et 5, on remplace un système d'aspiration buccal qui entraîne des risques de contamination par introduction de salive avec le milieu à récolter par des moyens d'aspiration légers, maniables et non contaminants et grâce à l'emploi d'un appareil à batterie rechargeable, on supprime l'utilisation d'une pompe à haute tension connectée au secteur.

Dans les modes de réalisation qui viennent d'être décrits, l'invention est considérée comme étant appliquée à la collecte de liquide séminal.

On peut cependant envisager d'utiliser le dispositif et l'appareil collecteur de l'invention pour collecter d'autres liquides visqueux.

## Revendications

1. Dispositif collecteur d'un liquide visqueux tel qu'un liquide séminal comprenant un récipient collecteur (1;25;36) sur lequel est monté un embout (2;30) pourvu d'une canule (3) d'aspiration du liquide sous l'effet d'une dépression, caractérisé en ce qu'il comporte en outre un manchon adaptateur (5) interposé entre le récipient (1;25;36) et l'embout (2;30), ledit manchon comprenant un filtre (11) en communication avec l'embout (2;30) d'une part et avec le récipient collecteur (1;25;36) d'autre part et un canal d'aspiration (21) destiné à être relié à une source de dépression pour engendrer dans le récipient une dépression destinée à provoquer l'aspiration du liquide à travers ledit filtre (11).

2. Dispositif collecteur suivant la revendication 1, caractérisé en ce que le manchon adaptateur (5) interposé entre le récipient (1;25;36) et l'embout (2;30), comprend une chambre (10) en communication avec l'embout (2;30) dans laquelle est disposé le filtre (11) pour le liquide, un conduit (16) d'aspiration du liquide comprenant une première portion d'extrémité évasée (17) débouchant sur le filtre (11) et une seconde portion d'extrémité (18) débouchant dans le récipient par une saillie (19) s'étendant axialement au-delà de l'extrémité (20) du manchon tournée vers le récipient et un canal d'aspiration (21) destiné à être relié à une source de dépression et aboutissant à l'extrémité (20) du manchon tournée vers le récipient pour engendrer dans le récipient (1;25;36) une dépression destinée à provoquer l'aspiration du liquide à travers ledit filtre (11).

3. Dispositif collecteur suivant l'une des revendications 1 et 2, caractérisé en ce que le récipient collecteur est un tube à essai (1) et le manchon adaptateur (5) comporte une portion (8) engagée dans le tube à essai pourvue de nervures annulaires (9) de sertissage inclinées dans le sens de l'engagement du manchon adaptateur (5) dans le tube à essai.

4. Dispositif collecteur suivant l'une des revendications 1 et 2, caractérisé en ce que le récipient collecteur est un Erlenmeyer (25) et le manchon adaptateur (5) comporte une portion engagée dans le col (26) de l'Erlenmeyer comprenant une portion (7) de surface latérale tronconique et une portion (8) pourvue de nervures annulaires (9), au moins un joint torique d'étanchéité (27) étant monté sur ladite portion (8) du manchon adaptateur dans une gorge définie entre deux des nervures (9).

5. Dispositif collecteur suivant l'une des revendications 1 et 2, caractérisé en ce que le récipient collecteur est un récipient tubulaire de faible section (36) et en ce qu'il comporte un manchon souple (35) de liaison dudit récipient tubulaire (36) s'adaptant de façon étanche d'une part sur une portion (8) du manchon adaptateur (5) tournée vers ledit récipient (36) et d'autre part sur le pourtour dudit récipient (36, la saillie (19) par laquelle le conduit d'aspiration (16) du manchon adaptateur (5) débouche dans le récipient étant engagée avec un jeu (37) dans le col dudit récipient (36).

6. Dispositif collecteur suivant la revendication 5, caractérisé en ce que ledit récipient collecteur de faible section (36) est une ampoule précassée.

7. Dispositif collecteur suivant l'une des revendications 1 à 6, caractérisé en ce que l'embout (2) pourvu de la canule d'aspiration du liquide est un capuchon en matière souple monté sur une paroi extérieure latérale (6) du manchon adaptateur pourvue d'une collerette de butée (7).

8. Dispositif collecteur suivant la revendication 7, caractérisé en ce qu'il comporte en outre une douille (12) de fixation du filtre (11) qui comporte une jupe (13) en contact avec la surface latérale de la chambre (10) du manchon adaptateur (5) et appliquant le bord du filtre contre le fond de ladite chambre et un rebord (14) définissant intérieurement un réceptacle évasé (15) pour le liquide séminal et s'étendant immédiatement au-dessous de l'extrémité de la canule d'aspiration (3) engagée dans le capuchon souple (2), le réceptacle aboutissant à un étranglement (15a) qui communique à son tour avec une chambre (15b) assurant la répartition du liquide sur la totalité de la surface utile du filtre.

9. Dispositif collecteur suivant l'une des revendications 1 à 6, caractérisé en ce que l'embout (30) pourvu de la canule d'aspiration du liquide séminal est un capuchon rigide comportant une jupe (34) engagée dans la chambre (10) du manchon adaptateur et assurant le maintien du filtre (11), ledit capuchon rigide comprenant un canal étroit et axial (32) dans lequel est engagée la canule d'aspiration (3), ledit canal axial formant étranglement et débouchant dans une chambre tronconique (33) située juste au-dessus du filtre (11) et assurant la répartition du liquide sur la totalité de la surface utile du filtre.

10. Dispositif collecteur suivant l'une des revendications 1 à 7, caractérisé en ce que le filtre (11) est fixé dans la chambre (10) du manchon adaptateur (5) par collage ou soudage.

11. Dispositif collecteur suivant l'une des revendications 1 à 10, caractérisé en ce que le filtre (11) est un filtre à maille très fine, monofil, non mouillable dont la dimension est adaptée au produit à filtrer.

12. Dispositif collecteur suivant la revendication 11, caractérisé en ce que le filtre (11) est en polyester ou en polyamide non toxique, à maille inférieure à 100µm.

13. Appareil collecteur d'un liquide visqueux tel qu'un liquide séminal, caractérisé en ce qu'il comporte un dispositif collecteur de liquide suivant l'une des revendications 1 à 3 et 5 à 12, le dispositif collecteur étant engagé de façon amovible dans un boîtier (40) au moyen d'une bague d'adaptation (44) fixée dans une paroi (41) du boîtier et comportant des moyens (58,60,61,62) de liaison étanche du canal d'aspiration (21) du dispositif collecteur avec une source de dépression, comprenant une pompe (47) et un moteur électrique (46) d'actionnement de celle-ci également contenus dans le boîtier (40), le moteur électrique étant relié à une batterie d'alimentation (45) tandis que la pompe (47) est reliée par des conduits (48,51) à la bague d'adaptation (44).

14. Appareil collecteur suivant la revendication 13, caractérisé en ce que la batterie (45) est une batterie rechargeable et en ce qu'il comprend une prise (52) de connexion de la batterie à un chargeur de batterie.

15. Appareil collecteur suivant l'une des revendications 13 et 14, caractérisé en ce que les moyens de liaison étanche du canal d'aspiration (21) du dispositif collecteur avec la source de dépression (46,47) comprennent des joints toriques d'étanchéité (58,60) portés par la bague d'adaptation (44), une chambre annulaire (61) ménagée dans la bague entre les joints toriques et communiquant avec le canal d'aspiration (21) du manchon adaptateur (5) du dispositif collecteur monté dans le boîtier (40), la bague comprenant en outre un canal d'aspiration (62) débouchant dans la chambre annulaire (61) et relié directement à un conduit (51) de liaison avec le récipient décanteur (50) interposé entre la bague d'adaptation et la pompe (47).

16. Appareil collecteur suivant l'une des revendications 13 à 15, caractérisé en ce qu'il comporte en outre une poignée (43) en forme de crosse fixée au corps (40) du boîtier, ladite poignée comportant une branche libre (54) portant un interrupteur (55) de commande du moteur (46) d'entraînement de la pompe à dépression (47), ledit interrupteur pouvant être actionné par les doigts de la main de l'utilisateur alors que le dos de cette main est en contact avec le bord (43) du boîtier (40).

## Claims

1. Collection device for a viscous liquid such as a seminal liquid comprising a collection container (1; 25; 36) on which is mounted a cap (2; 30) provided with a cannula (3) for suction of the liquid under the effect of a vacuum, characterised in that it also comprises an adaptor sleeve (5) interposed between the container (1; 25; 36) and the cap (2; 30), said sleeve comprising a filter (11) connected to the cap (2; 30) on one side and to the collection container (1; 25; 36) on the other side and a suction channel (21) intended to be connected to a source of vacuum in order to produce in the container a vacuum intended to cause the suction of the liquid through said filter (11).

2. Collection device according to Claim 1, characterised in that the adaptor sleeve (5) interposed between the container (1; 25; 36) and the cap (2; 30), comprises a chamber (10) communicating with the cap (2; 30) in which the filter (11) for the liquid is disposed, a pipe (16) for the suction of the liquid comprising a first flared end portion (17) opening out at the filter (11) and a second end portion (18) opening out in the container through a projection (19) extending axially beyond the end (20) of the sleeve facing the container and a suction channel (21) intended to be connected to a source of vacuum and terminating at the end (20) of the sleeve facing the container in order to produce in the container (1; 25; 36) a vacuum intended to cause the auction of the liquid through said filter (11).

3. Collection device according to one of Claims 1 and 2, characterised in that the collection container is a test tube (1) and the adaptor sleeve (5) comprises a portion (8) engaged in the test tube provided with annular seating ribs (9) inclined in the direction of engagement of the adaptor sleeve (5) in the test tube.

4. Collection device according to one of Claims 1 and 2, characterised in that the collection container is an Erlenmeyer flask (25) and the adaptor sleeve (5) comprises a portion engaged in the neck (26) of the Erlenmeyer flask comprising a portion (7) of frustoconical lateral surface and a portion (8) provided with annular ribs (9), at least one O-ring (27) being mounted on said portion (8) of the adaptor sleeve in a groove defined between two of the ribs (9).

5. Collection device according to one of Claims 1 and 2, characterised in that the collection container is a tubular container of small section (36) and in that it comprises a flexible sleeve (35) for the connection of said tubular container (36) fitting in a sealed manner on the one hand on a portion (8) of the adaptor sleeve (5) facing said container (36) and on the other hand on the periphery of said container (36), the projection (19) by which the suction tuba (16) of the adaptor sleeve (5) opens into the container being engaged with a clearance (37) in the neck of said container (36).

6. Collection device according to Claim 5, characterised in that said collection container of small section (36) is a prebroken ampoule.

7. Collection device according to one of Claims 1 to 6, characterised in that the cap (2) provided with the cannula for the suction of liquid is a cover of flexible material mounted on an outer side wall (6) of the adaptor sleeve provided with an abutment flange (7).

8. Collection device according to Claim 7, characterised in that it also comprises a bush (12) for fixing the filter (11), which comprises a skirt (13) in contact with the lateral surface of the chamber (10) of the adaptor sleeve (5) and pressing the edge of the filter against the bottom of said chamber and a rim (14) internally defining a flared receptacle (15) for the seminal liquid and extending immediately below the end of the suction cannula (3) engaged in the flexible cover (2), the receptacle ending at a constriction (15a) which in turn communicates with a chamber (15b) ensuring the distribution of the liquid over the entire useful surface of the filter.

9. Collection device according to one of Claims 1 to 6, characterised in that the cap (30) provided with the cannula for the suction of the seminal liquid is a rigid cover comprising a skirt (34) engaged in the chamber (10) of the adaptor sleeve and ensuring the retention of the filter (11), said rigid cover comprising a narrow and axial channel (32) in which is engaged the suction cannula (3), said axial channel forming a constriction and opening out in a frustoconical chamber (33) situated just above the filter (11) and ensuring the distribution of the liquid over the entire useful surface of the filter.

10. Collection device according to one of Claims 1 to 7, characterised in that the filter (11) is fixed in the chamber (10) of the adaptor sleeve (5) by sticking or welding.

11. Collection device according to one of Claims 1 to 10, characterised in that the filter (11) is a non-wettable, monofilament filter with a very fine mesh, whereof the dimensions are adapted to the product to be filtered.

12. Collection device according to Claim 11, characterised in that the filter (11) is made of non-toxic polyester or polyamide, with a mesh less than 100 µm.

13. Collection apparatus for a viscous liquid such as a seminal liquid, characterised in that it comprises a liquid collection device according to one of Claims 1 to 3 and 5 to 12, the collection device being engaged in a removable manner in a casing (40) by means of a fitting ring (44) fixed in a wall (41) of the casing and comprising means (58, 60, 61, 62) for the hermetic connection of the suction channel (21) of the collection device to a source of vacuum, comprising a pump (47) and an electric motor (46) for actuating the latter, which are also contained in the casing (40), the electric motor being connected to a power supply battery (45) whereas the pump (47) is connected by tubes (48, 51) to the fitting ring (44).

14. Collection apparatus according to Claim 13, characterised in that the battery (45) is a rechargeable battery and in that it comprises a plug (52) for connecting the battery to a battery charger.

15. Collection apparatus according to one of Claims 13 and 14, characterised in that the means for the hermetic connection of the suction channel (21) of the collection device to the source of vacuum (46, 47) comprise O-rings (58, 60) carried by the fitting ring (44), an annular chamber (61) provided in the ring between the O-rings and communicating with the suction channel (21) of the adaptor sleeve (5) of the collection device mounted in the casing (40), the ring furthermore comprising a suction channel (62) opening into the annular chamber (61) and connected directly to a tube (51) for connection to the decanting container (50) interposed between the fitting ring and the pump (47).

16. Collection apparatus according to one of Claims 13 to 15, characterised in that it also comprises a handle (43) in the shape of a crook fixed to the body (40) of the casing, said handle comprising a free side (54) supporting a switch (55) for controlling the motor (46) for driving the vacuum pump (47), said switch being able to be actuated by the fingers of the user's hand whereas the back of this hand is in contact with the edge (43) of the casing (40).

## Patentansprüche

1. Sammelvorrichtung für eine viskose Flüssigkeit, wie einer Seminalflüssigkeit mit einem Sammelbehälter (1;25;36), auf welchem ein Ansatzstück (2;30) angebracht ist, welches mit einer Ansaugkanüle (3) für die Flüssigkeit unter der Wirkung von Unterdruck ausgestattet ist, dadurch gekennzeichnet, daß sie außerdem einen zwischen den Behälter (1;25;36) und das Ansatzstück (2;30) gesetzten Anpaßstutzen (5) aufweist, wobei der Stutzen einen Filter (11) aufweist, welcher einerseits mit dem Ansatzstück (2;30) und andererseits mit dem Sammelbehälter (1;25;36) verbunden ist, und einen Ansaugkanal (21), welcher dafür bestimmt ist, mit einer Unterdruckquelle verbunden zu sein, um in dem Behälter einen Unterdruck zu erzeugen, der dafür bestimmt ist, die Ansaugung der Flüssigkeit durch den Filter (11) zu bewirken.

2. Sammelvorrichtung gemäß Anspruch 1, dadurch gekennzeichnet, daß der zwischen den Behälter (1;25;36) und das Ansatzstück (2;30) gesetzte Anpaßstutzen (5) eine Kammer (10), welche mit dem Ansatzstück (2;30) verbunden und in welcher der Filter (11) für die Flüssigkeit angeordnet ist, eine Ansaugleitung (16) für die Flüssigkeit, welche einen ersten konisch erweiterten Endbereich (17), der in den Filter (11) mündet, und einen zweiten Endbereich (18) aufweist, welcher in den Behälter durch einen Vorsprung (19) mündet, welcher sich axial über das Ende (20) des zu dem Behälter hin gedrehten Stutzens erstreckt, und einen Ansaugkanal (21) aufweist, welcher dafür bestimmt ist, mit einer Unterdruckquelle verbunden zu werden, und welcher an das Ende (20) des zu dem Behälter hin gedrehten Stutzens anstößt, um in dem Behälter (1;25;36) einen Unterdruck zu erzeugen, welcher dafür bestimmt ist, die Ansaugung der Flüssigkeit durch den Filter (11) zu bewirken.

3. Sammelvorrichtung gemäß einem der Ansprüche 1 und 2, dadurch gekennzeichnet, daß der Sammelbehälter ein Reagenzglas (1) ist und der Anpaßstutzen (5) einen in das Reagenzglas eingeführten Bereich (8) aufweist, welcher mit ringförmigen Rippen (9) zum Festziehen versehen ist, welche in dem Einführsinn des Anpaßstutzens (5) in das Reagenzglas schräg gestellt sind.

4. Sammelvorrichtung gemäß einem der Ansprüche 1 und 2, dadurch gekennzeichnet, daß der Sammelbehälter ein Erlenmeyerkolben (25) ist und der Anpaßstutzen (5) einen in den Hals (26) des Erlenmeyerkolbens eingeführten Bereich mit einem kegelstumpfartigen Seitenflächenbereich (7) und einem mit ringförmigen Rippen (9) versehenen Bereich (8) aufweist, wobei wenigstens eine torische Dichtungsverbindung (27) auf dem Bereich (8) des Anpaßstutzens in einer zwischen zwei der Rippen (9) definierten Rille angebracht ist.

5. Sammelvorrichtung gemäß einem der Ansprüche 1 und 2, dadurch gekennzeichnet, daß der Sammelbehälter ein röhrenförmiger Behälter mit kleinem Querschnitt (36) ist, und daß sie einen flexiblen Stutzen (35) zur Verbindung mit dem röhrenförmigen Behälter (36) aufweist, welcher sich abdichtend einerseits auf einen Bereich (8) des zu dem Behälter (36) hin gedrehten Anpaßstutzens (5) und andererseits auf den Umfang des Behälters (36) schmiegt, wobei der Vorsprung (19), durch welchen die Ansaugleitung (16) des Anpaßstutzens (5) in den Behälter mündet, mit einem Spiel (37) in den Hals des Behälters (36) eingeführt ist.

6. Sammelvorrichtung gemäß Anspruch 5, dadurch gekennzeichnet, daß der Sammelbehälter (36) mit kleinem Querschnitt eine vorgeöffnete Ampulle ist.

7. Sammelvorrichtung gemäß einem der Ansprüche 1 bis 6, dadurch gekennzeichnet, daß das mit der Ansaugkanüle für die Flüssigkeit versehene Ansatzstück (2) eine Verschlußkappe aus flexiblem Material ist, welche auf einer äußeren Seitenwand (6) des mit einem Anschlagkragen (7) versehenen Anpaßstutzens angebracht ist.

8. Sammelvorrichtung gemäß Anspruch 7, dadurch gekennzeichnet, daß sie außerdem einen Befestigungsring (12) für den Filter (11), welcher eine Verkleidung (13) aufweist, die im Kontakt mit der Seitenfläche der Kammer (10) des Anpaßstutzens (5) ist, und welche den Rand des Filters gegen den Boden der Kammer drückt, und einen Umschlag (14) aufweist, welcher in seinem Inneren einen konisch erweiterten Behälter (15) für die Seminalflüssigkeit definiert und sich unmittelbar unterhalb des Endes der in die flexible Verschlußkappe (2) eingeführten Ansaugkanüle (3) erstreckt, wobei der Behälter an eine Einschnürung (15a) anstößt, welche wiederum mit einer Kammer (15b) verbunden ist, welche die Verteilung der Flüssigkeit über die Gesamtheit der Nutzfläche des Filters gewährleistet.

9. Sammelvorrichtung gemäß einem der Ansprüche 1 bis 6, dadurch gekennzeichnet, daß das mit der Ansaugkanüle für die Seminalflüssigkeit versehene Ansatzstück (30) eine steife Verschlußkappe mit einer Verkleidung (34) ist, welche in die Kammer (10) des Anpaßstutzens eingeführt ist und das Halten des Filters (11) gewährleistet, wobei die steife Verschlußkappe einen engen und axialen Kanal (32) aufweist, in welchen die Ansaugkanüle (3) eingeführt ist, wobei der axiale Kanal eine Einschnürung bildet und in eine kegelstumpfartige Kammer (33) mündet, welche direkt oberhalb des Filters (11) angeordnet ist und die Verteilung der Flüssigkeit über die Gesamtheit der Nutzfläche des Filters gewährleistet.

10. Sammelvorrichtung gemäß einem der Ansprüche 1 bis 7, dadurch gekennzeichnet, daß der Filter (11) in der Kammer (10) des Anpaßstutzens (5) durch Kleben oder Schweißen befestigt ist.

11. Sammelvorrichtung gemäß einem der Ansprüche 1 bis 10, dadurch gekennzeichnet, daß der Filter (11) ein monofiler, nicht benetzbarer Filter mit sehr feinen Poren ist, deren Größe dem zu filtrierenden Produkt angepaßt ist.

12. Sammelvorrichtung gemäß Anspruch 11, dadurch gekennzeichnet, daß der Filter (11) aus Polyester oder aus nicht toxischem Polyamid ist, mit Poren, welche kleiner als 100 µm sind.

13. Sammelgerät für eine viskose Flüssigkeit wie eine Seminalflüssigkeit, dadurch gekennzeichnet, daß es eine Flüssigkeitssammelvorrichtung gemäß einem der Ansprüche 1 bis 3 und 5 bis 12 aufweist, wobei die Sammelvorrichtung herausnehmbar in einem Gehäuse (40) mittels eines Anpaßrings (44) angebracht ist, welcher in einer Wand (41) des Gehäuses (40) befestigt ist und Vorrichtungen (58,60,61,62) zur dichten Verbindung des Ansaugkanals (21) der Sammelvorrichtung mit einer Unterdruckquelle aufweist, welche eine Pumpe (47) und einen elektrischen Antriebsmotor (46) für diese aufweisen, die auch in dem Gehäuse (40) enthalten sind, wobei der elektrische Motor mit einer Versorgungsbatterie (45) verbunden ist, während die Pumpe (47) über Leitungen (48,51) mit dem Anpaßring (44) verbunden ist.

14. Sammelgerät gemäß Anspruch 13, dadurch gekennzeichnet, daß die Batterie (45) eine aufladbare Batterie ist, und daß es eine Anschlußstelle (52) der Batterie an ein Batterieladegerät aufweist.

15. Sammelgerät gemäß einem der Ansprüche 13 und 14, dadurch gekennzeichnet, daß die Vorrichtungen zur dichten Verbindung des Ansaugkanals (21) der Sammelvorrichtung mit der Unterdruckquelle (46,47) torische Dichtungsverbindungen (58,60), welche von dem Anpaßring (44) getragen werden, und eine ringfömige Kammer (61) aufweisen, welche in dem Ring zwischen den torischen Verbindungsstellen angeordnet ist und mit dem Ansaugkanal (21) des Anpaßstutzens (5) der in dem Gehäuse (40) angebrachten Sammelvorrichtung verbunden ist, wobei der Ring außerdem einen Ansaugkanal (62) aufweist, welcher in die ringförmige Kammer (61) mündet und direkt mit einer Leitung (51) zur Verbindung mit dem zwischen den Anpaßring und die Pumpe (47) zwischengesetzten Dekantierbehälter (50) verbunden ist.

16. Sammelgerät gemäß einem der Ansprüche 13 bis 15, dadurch gekennzeichnet, daß es außerdem einen Griff (43) in Hakenform aufweist, welcher an dem Körper (40) des Gehäuses befestigt ist, wobei der Griff einen freien Arm (54) aufweist, der einen Steuerschalter (55) für den Antriebsmotor (46) der Unterdruckpumpe (47) trägt, wobei der Schalter von den Fingern der Hand des Benutzers betätigt werden kann, während der Rücken dieser Hand sich im Kontakt mit dem Rand (43) des Gehäuses (40) befindet.
